Europäisches Patentamt

European Patent Office

Office européen des brevets

(11) Publication number: **0 027 834**
A1

# EUROPEAN PATENT APPLICATION

(21) Application number: **79200611.6**

(22) Date of filing: **24.10.79**

(51) Int. Cl.³: **C 12 N 9/99,** C 12 N 9/58,
A 23 C 19/032

(43) Date of publication of application: **06.05.81**
Bulletin 81/18

(84) Designated Contracting States: **NL**

(71) Applicant: **RAPIDASE B.V., Wateringseweg 1 P.O.-box 1,
NL-2600 MA Delft (NL)**

(72) Inventor: **Colein, Bernard Albert Victor, Rue Leo
Lagrange 19, F-59263 Houplin-Ancoisne (FR)**
Inventor: **Delecourt, Robert Louis Felix, Rue Roger
Bouvry 26, F-59113 Séclin (FR)**

(74) Representative: **Van der Straaten, Jan Anthony et al, c/o
GIST-BROCADES N.V. Patents and Trademarks
Department Wateringseweg 1 P.O. Box 1, NL-2600 MA
Delft (NL)**

(54) Thermally sensitive microbial rennet, a process for its preparation and its application for cheese preparation.

(57) Process comprising the cultivation of microbial rennet producing microorganism and treatment of the microbial rennet producing microorganism and treatment of the microbial rennin containing liquid, obtained after recovery of the fermentation broth, with an modifying agent, which enhances the thermal instability, while retaining the milk coagulating activity, of the microbial rennet in the residual liquids, obtained after cheese preparation, to be used for further food processing, as well as the use of such obtained thermally sensitive microbial rennin for the cheese preparation and the use of the obtained whey for further food processing.

Rapidase B.V. at Delft.

Thermally sensitive microbial rennet, a process for its preparation and its application for cheese preparation.

The invention relates to a thermally sensitive microbial rennet, to a process for the preparation of such thermally sensitive microbial rennet as well as the use of such obtained thermally sensitive microbial rennet for the cheese preparation and the use of the obtained whey for further food processing.

It is well known to prepare various types of milk coagulating enzymes, further to be mentioned microbial rennet, by cultivation of certain Mucor miehei strains in suitably selected culture mediums and recovering the microbial rennet from the fermentation broth.

Such processes are disclosed in e.g. U.S. patent no. 3988207 and U.S. patent no. 4136201.

Moreover from U.S. patent 4136201 and more particularly U.S. patent 3616233 it was known to prepare microbial rennet, which is substantially free of lipase and/or esterase.

The so obtained milk coagulating enzymes are successfully applied for the preparation of various types of cheese, giving rise to a major byproduct called whey.

Whey has been looked upon as a potential starting material for processes to prepare a variety of food stuffs.

Such processes essentially comprise the pasteurization and spray drying of whey in order to obtain a powder, which is very rich in valuable ingredients of food stuffs. This powder is subsequently mixed with milk or liquids derived from milk in order to obtain homogeneous, attractively looking finally desired products.

A well recognized problem of this processing of the whey is the occurrence therein of residual, still active microbial rennet, which is still able to coagulate milk or liquids derived from milk and which actually prevents the preparation of the desired homogeneous, attractive looking products.

It was found that whey containing calf rennet, did not give such problems, due to a relative greater thermal instability of such rennet, and that the hereinbefore mentioned problems, connected with the use of microbial rennet containing whey, could only be eliminated by a previous heating step to destruct residual microbial rennet, using relatively high temperatures; such procedure, however, gives rise to unattractively colored products.

On the other hand, from e.g. U.S. patent no. 4086139 was known the selective inactivation of amylase in a mixture of protease and amylase by contacting the mixture with hypochlorite or chlorite ions as oxidizing agent in an amount which is effective for the inactivation of the amylase to a substantially greater extent than the protease.

From U.S. patent no. 4087330 was known the regeneration of a spent immobilised enzyme composition by treating it with aqueous sodium hypochlorite, the spent immobilised enzyme composition comprising a porous, high surface area, insoluble inorganic support material having contaminants associated with it. All contaminants are removed by this regeneration process whereafter the support material is reacted with an enzyme, such as glucose isome rase, glucoamylase or lactase, in aqueous solution. However, this process is primarily used for the removal of the enzymeresidues, which have become inactive after their use.

Moreover the Belgian patent no. 848175, discloses the chemical fixation of organic substances containing a sugar residue to a support containing at least one reactive amino group, by the oxidation of at least one hydroxymethyl group in the sugar residue to an aldehyde group, and reacting the resulting aldehyde group with amino groups carried by side chains fixed to an insoluble solid support. This process can be used to immobilise glycoproteins, such as enzymes, while as oxidizing agent e.g. a sodium iodite is used.

One of the objects of this invention is to provide a novel thermally sensitive microbial rennet which has retained the major part of its milk coagulating activity at the ordinary temperature of cheese-making.

With the term "thermally sensitive" is meant to indicate that the microbial rennet looses its milk coagulating activities almost completely at temperatures above 60°C.

Another object is to provide a novel process for producing such a thermally sensitive microbial rennet.

A further object is to provide an improved process for the preparation of cheese, using the novel thermally sensitive microbial rennet.

An additional object is to provide an improved method for the preparation of food stuffs derived from the whey, which is the residue of the cheese preparation, using the novel, thermally sensitive microbial rennet.

The present invention relates to a novel, thermally sensitive microbial rennet, which has retained the major part of its milk coagulating activity, and more specifically relates to a thermally sensitive microbial rennet, which becomes deactivated under normal process conditions for the preparation of food stuffs, derived from whey, i.e. it looses its milk coagulating activities almost completely at temperatures above 60°C.

As a result of research and experimentation it was surprisingly found that novel thermally sensitive microbial rennets can be prepared by a process, comprising the treatment of a microbial rennet containing liquid with a chemically modifying agent.

The process may be applied as well to a filtrate, derived by recovery of the fermentation broth, obtained by cultivation of a _Mucor_ strain in a suitable nutrient medium and preferably a _Mucor miehei_ strain, or to a more or less concentrated filtrate, obtained by evaporation under reduced pressure or by ultrafiltration of such filtrate.

As suitable chemically modifying agents for the presently proposed treatment of the microbial rennet compounds such as salts of trichlorocyanuric acid, periodic acid, peracetic acid, hypochloric acid, chlorid acid and iodic acid,or mixtures thereof,can be used. Preferably the sodium, potassium or calcium salts of these acids and more especially sodium hypochlorite are used.

The modifying process is preferably carried out in an aqueous solution, having an activity corresponding with 0.5-4 g of active chlorine per liter of starting medium and more preferably 1.5-2 g of active chlorine per liter of microbial rennet containing medium.
According to a preferred embodiment of the invention the agents are added to the microbial rennet containing liquid, in the form of 5-20 ml of an aqueous stock solution, having an activity corresponding with 140-160 g of active chlorine per liter, per liter of microbial rennet containing medium.

The obtained degree of the loss of milk coagulating activity is found to increase with increasing concentration of hypochlorite and varies in the range of from 0-15% of the original activity.

The process is carried out at temperatures varying from the temperature of freezing the solution to room temperature, i.e. at temperatures in the range of from -10 to +25°C, whereby the higher temperatures in the range have been found to give rise to more inactivation, although such an increase has been found to be very weak.

The process can be carried out at pH values varying in the range of from 2 to 8 and more preferably in the range of from 2.5 to 5.

According to another feature of the invention, it was surprisingly found that as a result of the modifying reaction a thermally sensitive microbial rennet could be obtained, which was substantially free of any lipase and/or esterase activity, any of such activity, present in the microbial rennet containing starting liquid being completely deactivated by and as a result of the process.

Therefore, according to a preferred embodiment of the process of the present invention, the originally present lipase and/or esterase activity is eliminated by carrying out the process at a pH value of about 2.5 in order to eliminate the usual previous separate inactivation step for this accomponying lipase/esterase while the present process may succesfully be carried out at pH values in the range of from 4-5, when starting from microbial rennet containing solutions, which are substantially free of lipase and/or esterase.

The major part of the thermal instability of the desired microbial rennet can be obtained according to the process of the present invention within 1-15 minutes after mixing the modifying agent with the microbial rennet containing solution, while for obtaining the desired degree of thermal instability, total reaction times of up to 4 hours may be necessary, depending on the actual activity of the oxidizing agent, the working temperature and pH value.

During the process of the present invention, the coloration of the microbial rennet decreases significantly (10 to 25 %), while the finally obtained microbial rennet moreover has a more attractive appearance and smell as compared to the untreated microbial rennet.

It will be appreciated that these improved characteristics of the thermally sensitive microbial rennet represent an advantage inherent in the present invention.

Moreover, another advantage of the process of the present invention is that it can be carried out in rather simple equipment, e.g. in a usual tank with a stirrer and is consisting of rather simple measures at all.

In this connection it will be appreciated that the rate of the addition of the oxidizing agent has appeared to have only little influence or no influence at all on the loss of milk coagulating activity of the microbial rennet when subjected to the treatment according to the invention.

The thermally sensitive microbial rennet according to the present invention has a normal shelf life not essentially different from that of ordinairy untreated microbial rennets, when stored at pH values in the range of from 4.0 to 6.0 and preferably of from 4.5 to 5.5.

According to a preferred embodiment of the process of the present invention, it has appeared to be possible to obtain a

0027834

thermally sensitive microbial rennet which has lost about 5% of its original milk coagulating activity, by a treatment with 10-15 ml of a sodium hypochlorite solution, having an activity corresponding with about 150 g active chlorine/l, per liter of microbial rennet containing medium, i.e. fermentation liquor, at a pH between 3.7 and 5.0 and for a wide range of reaction times.

According to another preferred embodiment of the process it has appeared to be possible to prepare a thermally sensitive microbial rennet, which is substantially free of lipase and/or esterase activity, by treatment of a starting solution, which contains the normal amounts of lipase and/or esterase, with 5 ml of sodium hypochlorite solution per liter of microbial rennet containing medium at a pH of about 2.5.

It will be appreciated that the surprisingly obtained results of the rather simple process of the present invention could in no way be predicted by people skilled in the art, as it has appeared that not all conventional oxidizing agents could be used. E.g. hydrogen peroxide did not give any practical result.

As indicated hereinbefore, the process of the present invention may be as well applied to microbial rennet containing liquids, which are exhibiting lipase and/or esterase activity, as to those ones, the lipase and/or esterase activity of which has previously been substantially eliminated during a recovery process as described in U.S. patent 3,616,233, all relevant parts of which have to be considered as integrally inserted herein.

The microbial rennet activity is determined by the following analytical method.

The time of clotting a defined milk substrate is used to calculate the coagulating activity of the microbial rennet.

One soxhlet unit is the amount of rennet which is clotting one ml of milk substrate in 40 minutes at 35°C.

The substrate is prepared by dissolving 100 g of skimmed powdered milk in one liter of 0.2 g/l calcium chloride solution.

The measured time must be in the range of from 4 to 20 minutes.

The activity is calculated according to the following equation:

$$\frac{40 \times Vo}{t \times cxV} = \text{activity in soxhlet units}$$

Vo = volume of milk substrate

V  = volume of enzymatic solution

t  = clotting time in seconds

c  = concentration of enzyme in V ml.

For the <u>Mucor</u> <u>miehei</u> microbial rennet the same analysis is also realized at 63°C.

Due to its thermostability, the activity of <u>Mucor</u> <u>miehei</u> rennet at 63°C is about 170 percent of the activity at 35°C.

The evolution of the activity at 63°C is a direct measurement of the thermostability of the <u>Mucor</u> <u>miehei</u> rennet.

The esterase activity preferably is measured by a test on tributyrin, whereas the lipase activity preferably is measured by a test on an olive oil substrate.

These tests can be carried out according to the methods as described in column 2, lines 45-68, columns 3 and 4 and column 5, lines 1-41 of U.S. patent no. 4065580, the contents of which have to be considered as integrally inserted herein.

The following examples illustrate the practice of the invention and are not in any way to be considered as limiting to the spirit or scope of the invention.

### Example 1

One liter of a microbial rennet containing filtrate was obtained after fermentation with an isolate from <u>Mucor</u> <u>miehei</u> NRRL-A 13042 and recovery of the fermentation broth in the usual way, during which possibly present lipase and/or esterase activity is eliminated according to the method of US patent 3,616,233.

This filtrate has an activity of 112.00 Soxhlet units/ml at 35°C and of 193.00 units at 63°C. To this filtrate 10 ml of a sodium hypochlorite stock solution, having an activity corresponding with 150 g active chlorine per liter, were added under stirring in an open vessel.

The temperature of the mixture was kept at 7.5°C and the pH was 4.1.

After 4 hours processing, the activity of the microbial rennet was measured again. The respective activities are listed below.

clotting activity in Soxhlet units

|  | 35°C | | 63°C | |
|  | units/ml | percent | units/ml | percent |
|---|---|---|---|---|
| reference sample | 11 200 | 100 | 19 300 | 173 |
| processed sample | 10 400 | 93 | less than 100 | 0 |

## Example 2

To one liter of a microbial rennet containing solution, obtained by filtration of the fermentation broth from a Mucor miehei fermentation as produced according to example 1, followed by ultrafiltration and deactivation of lipase and/or esterase according to the method described in US patent 3,616,233, 10 ml of a sodiumhypochlorite solution, having an activity corresponding with 150 g/liter of active chlorine, were added under stirring and the liquid was processed in the same way as in example 1.

The clotting activities of the liquid before and after the treatment are listed below.

clotting activity in Soxhlet units

|  | 35°C | | 63°C | |
|  | units/ml | percent | units/ml | percent |
|---|---|---|---|---|
| reference sample | 224 000 | 100 | 400 000 | 180 |
| processed sample | 216 000 | 96 | less than 200 | 0 |

## Example 3

A filtered microbial rennet solution obtained from a Mucor miehei fermentation as produced according to example 1, is concentrated by ultrafiltration, while the esterase activity is not removed.

To one liter of such solution, 12 ml of sodium hypochlorite (having an activity corresponding with 153 g of active chlorine/ liter) were added under stirring at a temperature of 5°C and at pH 2.5. Stirring was continued during 4 hours and the clotting activity and the esterase activity were determined, as listed below.

clotting activity in Soxhlet units

|  | 35°C | | | 63°C | | esterase activity |
|  | units/ml | percent | | units/ml | percent | ea units |
|---|---|---|---|---|---|---|
| reference sample | 250 000 | 100 | | 425 000 | 170 | 15 ea |
| processed sample | 237 500 | 95 | | less than 200 | 0 | 0.2 ea |

From these values it can clearly be derived that the thermostability of microbial rennet has decreased while simultaneously esterase has been inactivated.

The microbial rennet obtained can be used to make cheddar cheese without developing any rancidity on aging.

The whey resulting from manufacturing cheese does not demonstrate any residual milk clotting activity when normally processed, i.e. when subjected to pasteurisation at 60°C and spray drying.

Claims:

1. A thermally sensitive microbial rennet, which has retained the major part of its milk coagulating activities at temperatures below 60°C.

2. A thermally sensitive microbial rennet, as claimed in claim 1, which is substantially free of lipase and/or esterase activity.

3. A thermally sensitive microbial rennet which has lost substantially all of its milk coagulating activity at temperatures above 63°C.

4. A process for the preparation of a thermally sensitive microbial rennet which comprised treatment of an aqueous microbial rennet containing liquid with an agent selected from the group consisting of the sodium, potassium and calcium salts of trichloro-cyanuric acid,per acetic acid, periodic acid, hypochloric acid, chloric acid and iodic acid.

5. A process as claimed in claim 4, in which said agent is sodium hypochlorite.

6. A process as claimed in claim 5, in which said agent is present in an activity, corresponding with 0.5-4 g of active chlorine per liter of microbial rennet containing medium, obtained by filtration of the fermentation broth.

7. A process as claimed in claim 6, in which said oxidizing agent is present in an activity, corresponding with 1.5-2 g of active chlorine per liter of microbial rennet containing medium.

8. A process as claimed in claim 4, in which the temperature is in the range of from -10°C to +25°C.

9. A process as claimed in claim 4, in which the pH is selected in the range from 2 to 8.

10. A process as claimed in claim 9, in which the pH is selected in the range from 2.5 to 3.

11. Process for the preparation of cheese, characterized in that a thermally sensitive microbial rennet of claim 1 is used.

12. Process for the preparation of food stuffs essentially derived from whey, resulting from the process of claim 11, and milk or a liquid derived from milk.

## DOCUMENTS CONSIDERED TO BE RELEVANT

| Category | Citation of document with indication, where appropriate, of relevant passages | Relevant to claim |
|---|---|---|
| X | BIOCHIMICA ET BIOPHYSICA ACTA, vol. 328, 1973, pages 52-60 Amsterdam, NL. P.A. McBRIDE-WARREN et al.: "Structural and functional determinants of Mucor Miehei protease. II. Circular dichroic studies on the native and periodate-oxidized enzyme" <br> * Page 56, paragraph 2; table 1 * | 1-4,11, 12 |
| X | FR - A - 1 401 474 (CHAS. PFIZER & CO.) <br> * Page 2, column 1, lines 47-48; example 1; abstract x * <br> & NL - A - 64 04895 | 1,3, 11,12 |
| X | JOURNAL OF DAIRY SCIENCE, vol. 57, no. 5, 1974, page 591, M52 G.A. SOMKUTI et al.: "Milk clotting protease from psychrophilic fungi" <br> * Whole article * | 1,3, 11,12 |
| X | CHEMICAL ABSTRACTS, vol. 76, no. 13, 27th March 1972, page 272, no. 71083j Columbus, Ohio, U.S.A. SU, YUAN-CHI et al.: "Milk-clotting enzymes from microorganisms. II. Purification and physicochemical properties of the enzymes and their application in cheese manufacture" <br> & CHUNG KUO NUNG YEH HUA HSUEH HUI CHIH 1971, 9(1-2), 80-91 <br> * Abstract * <br> ./. | 1,3, 11,12 |

**CLASSIFICATION OF THE APPLICATION (Int. Cl. 3)**

C 12 N 9/99
9/58
A 23 C 19/032

**TECHNICAL FIELDS SEARCHED (Int.Cl. 3)**

C 12 N 9/00
9/48
9/50
9/58
9/99
A 23 C 19/032

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 19-06-1980 | DESCAMPS |

EPO Form 1503.1 06.78

| Category | Citation of document with indication, where appropriate, of relevant passages | Relevant to claim | CLASSIFICATION OF THE APPLICATION (Int. Cl. 3) |
|---|---|---|---|
| **DOCUMENTS CONSIDERED TO BE RELEVANT** ||||
| X | CHEMICAL ABSTRACTS, vol. 87, no. 17, 24th October 1977, page 524, no. 132475q Columbus, Ohio, U.S.A. LIN, CHIN WEN et al.: "Studies on the manufacture of oriental type cheese - The proteolytic properties of Mucor javanicus" & CHUNG-KUO NUNG YEH HUA HSUEH HUI CHIH 1977, 15(1-2), 49-58 * Abstract * | 1,3, 11,12 | |
| | DE - A - 2 300 474 (SOCIETE DES PRODUITS NESTLE) * Page 10, lines 8-10 * | 1,3, 11,12 | TECHNICAL FIELDS SEARCHED (Int. Cl. 3) |
| P,E | NL - A - 79 00315 (MILES LAB.) * Page 6, line 5 - page 7, line 1; examples 1-7; claims 1,4,5, 19,20 * & FR - A - 2 426 696 | 1,3, 11,12 | |
| A | JOURNAL OF DAIRY SCIENCE, vol. 62, no. 3, March 1979, pages 373-377 R.K. THUNELL et al.: "Thermal Inactivation of Residual Milk Clotting Enzymes in Whey" | | |
| A | JOURNAL OF DAIRY SCIENCE, vol. 62, no. 8, August 1979, pages 1227-1232 D.B. HYSLOP et al.: "Heat Inactivation of Milk-Clotting Enzymes at Different pH" | | |